# EUROPÄISCHE PATENTSCHRIFT

(11) **EP 1 082 957 B1**
(45) Veröffentlichungstag und Bekanntmachung des Hinweises auf die Patenterteilung: **23.04.2003**
(21) Anmeldenummer: 99117762.7
(22) Anmeldetag: 09.09.1999
(51) Int. Cl.: A61K 9/00

(54) **Brausegranulat mit verzögerter Brausewirkung**
Effervescent granulate with delayed effervescence
Granulé effervescent avec effervescence retardée

(43) Veröffentlichungstag der Anmeldung: 14.03.2001
(73) Patentinhaber: Dr. Gergely & Co., 1053 Wien (AT)
(72) Erfinder: Gergely, Gerhard, Dr., 1053 Wien (AT); Gergely, Irmgard, 1053 Wien (AT); Gergely, Thomas, Dr., 1053 Wien (AT)
(74) Vertreter: Büchel, Kurt F.

(56) Entgegenhaltungen:
- EP-A- 0 233 839
- EP-A- 0 642 784
- GB-A- 1 270 781
- US-A- 4 093 710
- US-A- 4 678 661

## Beschreibung

Die Erfindung betrifft ein Brausegranulat mit verzögerter Brausewirkung nach dem Oberbegriff des Anspruches 1, insbesondere für die Darreichung in Sachets.

Anders als die bekannten Brausetabletten sind in Sachets abgepackte Brausegranulate sehr häufig schlecht handhabbar: beim Auftreffen der - zwecks Erzielung einer raschen Auflösung meist verhältnismäßig feinkörnigen - Bestandteile des Brausesystems, wie z. B. Zitronensäure und Natriumhydrogencarbonat, auf die Oberfläche der Flüssigkeit beginnen diese Bestandteile sofort unter Brausewirkung zu reagieren. Je nach spezifischem Gewicht der weiteren Bestandteile, seien es Hilfs- oder Wirkstoffe, werden diese entweder für einige Zeit an der Oberfläche gehalten oder sinken zu Boden und lösen sich dort schlecht auf. Dieser Effekt wird noch verstärkt, wenn das Ausleeren des Granulats aus einem handelsüblichen, meist nahezu quadratischen Sachet erfolgt, das daher eine verhältnismäßig breite Aufreißöffnung (gelegentlich in der Größenordnung der Flüssigkeitsoberfläche) aufweist. Die Brausebestandteile treffen dann in verhältnismäßig dünner Schicht auf einen entsprechend großen Teil der Flüssigkeitsoberfläche.

Die Erfindung hat sich daher die Aufgabe gestellt, ein gut und frei fließendes Brausegranulat mit verzögertem Brauseeffekt zu entwickeln. Dieses Granulat ist bedingt durch seine spezifische Verpackung konsumentenfreundlich zu handhaben und kann Vitamine, Mineralstoffe, Spurenelemente bzw. pharmazeutische Wirkstoffe sowie Süßstoffe und Geschmackstoffe enthalten. Angestrebt wurde eine einfache Herstellung, die es ermöglicht, das Produkt kostengünstig zu erzeugen, und Granulate zu erhalten, die ohne zu reagieren beim Einbringen in Wasser zu Boden fallen und erst nach einigen Sekunden am Boden ihre Brausewirkung entfalten, wobei eine Durchwirbelung der Flüssigkeit erfolgt, die die Auflösung von Wirkstoffen oder Vitaminen, Mineralstoffen und weiteren Inhaltsstoffen mit sehr geringen Mengen solcher verzögerter Brausemischungen erzielt.

Diese Aufgabe wird erfindungsgemäß durch die Maßnahmen des Anspruches 1 überraschend gut gelöst. Vorteilhafte Weiterbildungen der Erfindung sind in den Kennzeichen der abhängigen Ansprüche beschrieben.

Es hat sich gezeigt, daß die Passivierung und die grobe Kornstruktur der organischer Säuren, wie Zitronensäure, Weinsäure, etc. einen gewissen Effekt bringt, der bei weitem aber nicht ausreicht, um eine ausreichende Verzögerung der Brausewirkung zu erreichen.

Es ist daher notwendig, die gasabspaltenden Bestandteile der Carbonat-Phase des Brausesystems, nämlich die Alkali- und/oder Erdalkalicarbonate und/oder Alkalihydrogencarbonate, so zu verzögern, bzw. zu verarbeiten, daß der gewünschte Effekt, nämlich ein deutlich verzögertes Brauseverhalten beim Einbringen in Wasser und die Entfaltung der Brausewirkung vom Boden her erzielt werden kann.

Verzögernde Substanzen können im allgemeinen Zucker, Zuckeralkohole, bzw. Kolloide sein, die auf das alkalische, gasabspaltende Gemisch von Carbonaten und Bicarbonaten aufgebracht werden und nach dem Trocknen einen Verzögerungseffekt ergeben.

Eine besonders für dieses System geeignete Wirkung erzielt man aber mit Polyethylenglycolen, die durch die Zeit, in der sie selbst Wasser aufnehmen, und auch durch ihre reaktionsstörende Funktion gute Verzögerungseffekte des Brausens bewirken. Bevorzugt wird Polyethylenglycol 6000 eingesetzt; es können aber auch Mischungen von Polyethylenglycol 4000 mit Polyethylenglycol 10.000 Verwendung finden. Als Einsatzmenge hat sich - bezogen auf die gasabspaltenden Bestandteile - eine Menge von 5 - 15 Gew.% bewährt, um den gewünschten Effekt zu erzielen.
In die Schmelze des Polyethylenglycols können noch weitere Substanzen, wie Kolloide, z. B. feinst pulverisierter Guar Gum oder Xanthan, sowie auch eine mikronisierte Säurekomponente eingebracht werden. Das Einbringen einer mikronisierten, schwer löslichen Säurekomponente in die Schmelze, wie Fumarsäure oder Adipinsäure, bringt den Vorteil, daß zu stark agglomerierte Granulate der Alkalihydrogencarbonate und -carbonate bzw. der Erdalkalicarbonate sich gut lösen können. Dabei genügt ein Einsatz von 1 - 3 Gew.% bezogen auf die gasabspaltenden Bestandteile, bzw. 10 - 30% bezogen auf das eingesetzte Polyethylenglycol.

Man kann nun die gasabspaltenden Substanzen mit alkoholischen Lösungen von Polyethylenglycolen granulieren und anschließend trocknen. Diese Vorgehensweise zeigt bereits in Mischung mit einer mit Erdalkalicarbonat behandelten Zitronensäure einen entsprechend verzögerten Effekt des Brausens.

Eine besonders bevorzugte Vorgehensweise der Herstellung für die Erzielung einer Verzögerung ist, derartige gasabspaltende Carbonate und/oder Bicarbonate homogen zu durchmischen und sodann aufgeschmolzene Polyethylenglycole bei hoher Temperatur dem Gemisch langsam zuzuführen, bevorzugt einzutropfen. Da bei den hohen Temperaturen von etwa 120 - 160°C Polyethylenglycole längere Zeit brauchen um fest zu werden, ergibt sich genügend Zeit, während des Mischens die einzelnen Partikel mit einem Film von Polyethylenglycol zu überziehen. Hierbei ist es natürlich ratsam, eine grobe Körnung der Alkalibestandteile zu wählen und beispielsweise gekörntes Natriumcarbonat oder -hydrogencarbonat grob kristallin einzusetzen, weil solche Teilchen schneller absinken, wodurch ein verzögerter Brauseeffekt vom Boden her entsteht. Es eignen sich dafür Natriumhydrogencarbonat, Kaliumhydrogencarbonat, Natriumcarbonat, Kaliumcarbonat, Calciumcarbonat sowie Magnesiumcarbonat.

Für Natriumhydrogencarbonat hat es sich als vorteilhaft erwiesen, eine Körnung zu wählen, bei der die Haupt-Siebfraktion im Bereich von 0.1 - 0.4 mm liegt, bevorzugt in einem Mengenanteil von 50 - 85 %, besonders bevorzugt von 70 - 85%. Für das Kaliumhydrogencarbonat empfiehlt sich eine Korngröße <0,5 mm, wobei der Hauptanteil der Partikel allerdings über 0,1 mm liegen sollte. Infolge der schlechteren Löslichkeit des Natriumcarbonats kann dieses in einer feineren Qualität eingesetzt werden, z.B. mit einem Hauptanteil der Körnung zwischen 0,07 und 0,2 mm. Calciumcarbonat wird bevorzugt in einer Menge von bis zu 30 Gew.% der gasabspaltenden Bestandteile eingesetzt.

Eine weitere Verbesserung des Systems wird - wie bereits angeführt -, durch die entsprechende oberflächliche Passivierung der Säuren, bevorzugt mit Calcium- oder Magnesiumsalzen, erreicht, sodaß dadurch zusätzlich eine Verzögerung des Reaktionsbeginns erreicht werden kann.

Zum Beispiel kann für Wirkstoffe wie z.B. Paracetamol, Lactulose, N-Acetylcystein, Ranitidin, Pflanzenextrakte, Multivitamin und/oder Spurenelemente als Säurekomponente eine mit 1 - 10 Gew.% Calciumcarbonat behandelte organische Säure dienen, zu der wie bereits beschrieben die in ihrer Brausewirkung verzögerten Alkalihydrogencarbonate, Alkalicarbonate und/oder Erdalkalicarbonate zugemischt werden. Damit lässt sich einerseits der gewünschte verzögerte Brauseeffekt erzielen und andererseits auch die angestrebte Stabilität erreichen.

Für Produkte, die Spurenelemente enthalten sollen, ist im Prinzip die gleiche Vorgangsweise denkbar, da die erfindungsgemässe Zusammensetzung keiner zusätzlichen Maßnahme zur Einarbeitung der Spurenelemente bedarf.

Bei Calcium- und/oder Magnesiumprodukten kann die Behandlung der organischen Säure mit dem jeweiligen Erdalkalicarbonat bzw. -oxid erfolgen. Die zusätzlich einzubringende Menge an Calcium und/oder Magnesium hängt weitgehend von der gewünschten Dosierung mit Calcium- bzw. Magnesiumionen ab, wobei z.B. bei Calcium additiv granulierte oder gefällte Calciumcarbonate, die entsprechende Fließeigenschaften haben, in die alkalische Carbonat-Phase eingearbeitet werden können. Bei Magnesiumprodukten kann zur Erreichung der gewünschten Menge an Magnesiumionen entweder bis zu 30 Gew.% Magnesiumcarbonat auf die organische Säure aufgebracht werden oder z.B. auch Trimagnesiumdicitrat der Endmischung zugefügt werden.

Als Säurekomponente wird bevorzugt Zitronensäure eingesetzt, deren Haupt-Siebfraktion von 0.3 bis 0.7 mm Korngrösse 50 bis 95 Gew.% ausmacht. Besonders bevorzugt wird Zitronensäure Grieß verwendet, der eine relativ grobe Körnung besitzt, wobei die Körnung der hauptsiebfraktion (ca. 80 %) zwischen 0.3 und 0.6mm liegt. Grössere Körnungen verlangsamen die Reaktion unter Umständen zu stark. Es kann aber auch eine etwas feinere Zitronensäure, z.B. mit einer Haupt-Siebfraktion von 50 bis 95 Gew.% zwischen 0,2 und 0,4 mm, zum Einsatz kommen, um das gewünschte Produkt zu erhalten, wenn die Körner entsprechend passiviert sind. Welche Korngrösse bevorzugt eingesetzt wird, hängt letztendlich nicht nur von der Lösungsgeschwindigkeit der gewählten sauren bzw. gasabspaltenden Komponente ab, sondern auch von der Endformulierung und den in das Granulat inkorporierten sonstigen Wirkstoffen. Die Zitronensäure kann z.B. mit 1 bis 10 Gew.% Calciumcarbonat vermischt und mit einer Lösung versetzt werden, wodurch es zu einer oberflächlichen Passivierung und damit einerseits zur verzögerten Brause-Reaktion, andererseits zu einer Stabilitätserhöhung des Granulates kommt. Die Lösung, mit der die Mischung von Zitronensäure mit Calciumcarbonat (das z.B. eine Partikelgröße < 5µ aufweist, wobei die bevorzugte Größe bei 0.1-0.2µ liegt) behandelt wird, kann eine wässrige oder eine alkoholische oder eine alkoholisch-wässrige ZitronensäureLösung sein, die zwischen 40 und 80 Gew.% Zitronensäure in der Lösung enthält.

Es ist auch möglich, entsprechend dem Europäischen Patent Nr. 0272312 eine wässrige Puffergranulier-Lösung einzusetzen. Um den Brauseeffekt noch stärker zu verzögern, können auch Hydrokolloide eingesetzt werden. Diese können entweder nach dem Befeuchten der Mischung von organischer Säure mit Carbonat aufgebracht werden, wobei eine Menge von 1 bis 5 Gew.% zu bevorzugen ist. Die Hydrokolloide können aber auch in die Granulierlösung eingebracht werden, wobei dann geringere Mengen von 0,1 - 1 Gew.% erforderlich sind.
Als Hydrokolloide können Maltodextrine, Polyvinylpyrrolidon, Guargum, Gummi Arabicum sowie alle in Wasser, Ethanol oder in deren Mischungen löslichen Hydrokolloide eingesetzt werden.

Die Behandlung der organischen Säure kann nach allen gängigen, technologisch bekannten Verfahren durchgeführt werden, wie z.B. im Wirbelschicht-Trockner, in Vakuum-Granulatoren und auch in kontinuierlichen Reaktionssystemen. Die behandelte organische Säure kann gegebenenfalls entsprechend getrocknet werden, um eine optimale Stabilität zu gewährleisten.

Nach der Behandlung wird die getrocknete organische Säure auf 1 bis 2 mm Korngrösse gesiebt. Die so behandelte Säure wird in einer Endmischung mit der Carbonat-Phase sowie den Wirkstoffen, bzw. den Vitaminen und/oder Mineralstoffen und/oder Spurenelementen vermischt. Mit einer Zusatzmenge von Alkalicarbonaten bzw. -hydrogencarbonaten kann auch der gewünschte pH-Wert eingestellt werden.

Diese Grundmischung kann mit den gängigen, zugelassenen Süßstoffen, wie Aspartam, Natriumcyclamat, Saccharin-Natrium, Acesulfam, aber auch mit entsprechenden Aromen und Geschmacksstoffen versehen werden. Bei Bedarf können auch Füllstoffe, die in der Körnung dem Grundgranulat angepaßt sind, eingebracht werden, wie Zucker oder Zuckeralkohole, z.B. Mannit, Sorbit, Xylit u.dgl.; auch granulierte Maltodextrine sind als Füllstoffe einsetzbar.

Dieses System ist in der Lage, relativ große Mengen löslicher Wirkstoffe durch die vom Boden einsetzende Durchwirbelung aufzulösen.Es können z.B. 10 g Lactulose mit ca. 3 bis 4 g der Granulatmischung von der mit Calciumcarbonat überzogenen Zitronensäure in Kombination mit der Carbonat-Phase aufgelöst werden.

Dieses System lässt gute Auflösungseigenschaften auch für Pflanzenextrakt-Granulate erzielen, die sonst beim Einbringen in Wasser sehr stark zum Verklumpen und Verschleimen neigen. Die Pflanzenextrakte können je nach Eigenschaft entweder dem System nur zugemischt werden, wie z.B. Echinacea, Agni Casti, Efeu, Cimicifuga. Es empfiehlt sich jedoch, dabei noch eine zusätzliche Maßnahme der Behandlung des Extraktes mit Antischaummittel oder Benetzungsmittel durchzuführen, wodurch der Effekt des sich vom Boden her mit Verzögerung auflösenden Granulates und die Durchwirbelung verbessert wird.

Die Wirkstoffe können in verschiedenster Weise in das System integriert werden. Bei wasserlöslichen Wirkstoffen genügt meistens die Zumischung zu den Brausekomponenten. Geringe Wirkstoffmengen können - je nach ihrer Empfindlichkeit gegenüber Säuren oder alkalischen Komponenten - entweder in die Zitronensäurekomponente oder in die Carbonat-Phase eingebracht werden, um sie gleichmäßig im Granulat zu verteilen.

Auch für unlösliche Wirkstoffe, wie z.B. Paracetamol, zeigt das System durch seinen speziellen Brauseeffekt eine äußerst gute Suspensionswirkung.
Bei sehr schlecht zu benetzenden und großen Mengen an unlöslichem Wirkstoff kann es auch erforderlich sein, daß der Wirkstoff speziell behandelt werden muß und sodann in Form einer eigenen Phase zugefügt wird.

Für Vitamin-Produkte, wie Vitamin C oder Multivitamin-Produkte, kann eine Trockenmischung der behandelten Zitronensäure mit der Carbonat-Phase sowie dem Vitamin C und für Multivitamin-Produkte mit den Multivitaminen, hergestellt werden, die anschließend vorzugsweise in spezielle, länglich geformte Sachets abgefüllt wird.

Ein weiterer Vorteil des erfindungsgemässen Brausesystems bringt eine spezielle Verpackung in schlauchförmigen Langsachets. Die Sachets werden vorzugsweise in einer Breite von 10 bis 30 mm und einer Länge von 7 bis 20 cm ausgeführt. Die erforderliche Menge an Brausegranulat hängt von den entsprechend einzutragenden Wirkstoffen ab. Sind nur geringe Wirkstoffmengen aufzulösen, kann das Gewicht der Sachets beispielsweise ca. 1g betragen. Bei Mineralstoff-Brausesachets, die einen gewissen Gehalt z.B an Calcium oder Magnesium enthalten sollen, ist es erforderlich, dafür die entsprechenden Menge an Zitronensäure einzusetzen, um den gewünschten pH einzustellen, wodurch Mineralstoff-Brausesachets beispielsweise 4-5g schwer sein können. Der Vorteil dieser länglichen Sachets ist die konsumentenfreundlichere Handhabung und das schnellere Eindringen des Granulates in Wasser, Saft oder Mischdrinks, weil das Granulat in dünnem, konzentriertem Strahl auf die Oberfläche der Flüssigkeit trifft, infolge des verzögert eingestellten Brauseeffekts nicht sofort unter Brausewirkung reagiert, sondern die Oberfläche rasch durchdringt, auf den Boden des Glases fällt und erst dort zu brausen beginnt. Auch damit kann zusätzlich verhindert werden, daß das Brausegranulat bereits beim ersten Kontakt mit Wasser an der Oberfläche braust und das Granulat an der Oberfläche schwimmt, bevor es absinkt und den Brauseeffekt erzeugt.

Überraschenderweise hat sich gezeigt, daß mit einer derart behandelten Zitronensäure sowie mit der Carbonat-Phase sowohl der gewünschte retardierte Brauseeffekt als auch eine von den ICH-Richtlinien geforderte Stabilität erreicht werden kann. Andererseits trägt das länglich geformte Sachet wesentlich zur Applikation der Darreichungsform bei, indem das Granulat nicht flächig auf die Wasseroberfläche aufgebracht wird, sondern wie durch ein Rohr auf den Boden des Glases fällt, um erst von dort her den Brauseeffekt zu entwickeln. Beispielhafte Ausführungen der Erfindung sind in den folgenden Beispielen angeführt:

### Beispiel 1: Herstellung passivierter Zitronensäure:

Die Herstellung kann in einem Wirbelschichttrocker oder auch in einem Vakuumgranulator durchgeführt werden.

In einem Mischer werden 1940 Gew.teile Zitronensäure Grieß und 60 Gew.teile Calciumcarbonat eingebracht und homogen vermischt. Diese Mischung wird mit 20 Gew.teilen einer Vorreaktionslösung bestehend aus 13 Gew.teilen Zitronensäure, 3 Gew.teilen Calciumcarbonat und 15 Gew.teilen Wasser versehen und ca. 3 Minuten homogen verteilt. Anschließend werden noch 11 Gew.teile der Vorreaktionslösung zugefügt und 5 Minuten homogen verteilt, wobei eine teilweise Reaktion auf der Oberfläche des Zitronensäure-Grieß erfolgt. Diese befeuchtete Mischung wird in einen Wirbelschichttrockner eingebracht und bei 70°C bis zu einer Restfeuchtigkeit von 0,1 % getrocknet. Anschließend wird das Granulat auf eine Korngrösse von 1,0 - 2,0 mm gesiebt.

Bei der Herstellung in einem Vakuumgranulator wird in gleicher Weise vorgegangen, es empfiehlt sich jedoch, die Zitronensäure und das Calciumcarbonat vor Einbringen der Lösung bis auf 60°C zu erwärmen. Die Trocknung erfolgt im Vakuum auf 15 mbar bei 60°C.

### Beispiel 2: Herstellung einer reaktionsverzögerten Carbonat-Phase

In einen Mischer werden eingebracht: 1000 Gew.teile grob kristallines Natriumhydrogencarbonat und 200 Gew.teile Calciumcarbonat. Die beiden Rohstoffe werden homogen vermischt, und anschließend werden 120 Gew.teile einer auf 130°C erhitzten Schmelze von Polyethylenglycol unter Rühren ein-gebracht und homogen verteilt. Nach dem Abkühlen wird das Carbonat-Granulat auf eine Korngrösse von 1,6 mm gesiebt.

Als optimale Formulierung hinsichtlich sowohl der Erzielung einer Verzögerung, als auch turbulenter Brausewirkung hat sich erwiesen, wenn in die Schmelze von 120 Gew.teilen Polyethylenglycol weiters 20 Gew.teile mikronisierte Fumarsäure sowie 36 Gew.teile Xanthan Gummi und 2,4 Gew.teile Aerosil sowie gegebenenfalls 10 Gew.teile Miglyol 812® (ein pflanzliches Neutralöl) eingebracht werden. Nach dem Abkühlen wird das Carbonat-Granulat auf eine Korngrösse von 1,6 mm gesiebt.

Aus den Grundgranulaten der Beispiele 1 und 2 lassen sich verschiedenste Formulierungen herstellen, wie z.B.:

### Beispiel 3: Vitamin C Brausegranulat mit verzögerter Brausewirkung

besteht aus 1400 Gew.teilen passivierter Säurekomponente, 980 Gew.teilen der Carbonat-Phase und 180 Gew.teilen Ascorbinsäure, 934 Gew.teilen Sorbit sowie nach Bedarf Süßstoffen und Aroma. Eine Dosis von 3,6 g, gegebenenfalls in Langsachets abgefüllt, enthält 180 mg Ascorbinsäure und fällt beim Einbringen in Wasser zu Boden. Erst 2 bis 5 Sekunden danach beginnt das Granulat sprudelnd zu brausen und sich schliesslich vollständig aufzulösen.

### Beispiel 4: Multivitamin-Brausegranulat

In gleicher Weise kann ein Multivitamin-Brausegranulat hergestellt und gegebenenfalls in Langsachets abgefüllt werden, wobei zusätzlich entsprechend den Tagesempfehlungen Vitamine sowie Süßstoffe, gegebenenfalls auch Farbstoffe und Aroma, zugefügt werden.

### Beispiel 5: Reaktionsverzögertes Calcium-Brausegranulat, enthaltend 250 mg Calcium:

Auch in diesem Fall kann - wie im Beispiel 1 beschrieben -, die mit Calciumcarbonat passivierte Zitronensäure-Phase eingesetzt werden, wobei die zusätzlich erforderliche Menge an Calcium in der Carbonat-Phase enthalten ist. Es kann in diesem Fall entweder ein granuliertes Calciumcarbonat oder ein gefälltes Calciumcarbonat mit einer gröberen Kornstruktur Verwendung finden.

Das Carbonat-Granulat wird analog dem Beispiel 2 hergestellt. Es werden in einem Mischer 904 Gew.teile Natriumhydrogencarbonat grob kristallin mit 628 Gew.teilen eines granulierten 36%igen Calciumcarbonates homogen vermischt und darauf die Polyethylenglycol 6000-Schmelze mit einer Temperatur von 130°C unter Rühren eingebracht und homogen verteilt. Die Schmelze besteht aus 108,5 Gew.teilen Polyethylenglycol 6000, 2 Gew.teilen Aerosil 200, 32 Gew.teilen Keltrol F, 9 Gew.teilen Miglyol 812 ® und 18 Gew.teilen Fumarsäure. Nach dem Abkühlen wird das Granulat auf eine Korngrösse von 1,6 mm gesiebt.

Aus den Bestandteilen der passivierten Zitronensäure und der Carbonat-Phase kann ein Endprodukt hergestellt werden, bestehend aus 2000 Gew.teilen passivierter Zitronensäure, 1702 Gew.teilen der Carbonat-Phase, 689 Gew.teilen Sorbit, 6 Gew.teilen Saccharin-Natrium, 50 Gew.teilen Aroma Orange und 2 Gew.teilen Yellow Nr. 6 zur Farbgebung.

Eine Dosis von 4,5 g enthält 250 mg Calcium, die in ein Langsachet verpackt wird und beim Einbringen in Wasser eine entsprechend verzögerte Auflösung zeigt.

### Beispiel 6: Magnesium-Brausegranulat:

Der Zitronensäure-Grieß wird mit ca. 30 Gew.% Magnesiumcarbonat (bezogen auf den Zitronensäure-Grieß) behandelt. Die Passivierung der Zitronensäure kann ebenfalls entweder in einem Granulator, in einem Wirbelschicht-Trockner oder in einem Vakuumgranulator passiviert werden.

Es werden in einem Mischer oder Granulator 560 Gew.teile Zitronensäure Grieß mit 178 Gew.teilen Magnesiumcarbonat homogen vermischt und auf 60°C erwärmt. Diese Mischung wird mit 120 Gew.teilen einer 50%igen wässrigen Zitronensäure-Lösung benetzt und 10 Minuten lang homogen verteilt, wobei eine teilweise Reaktion und eine Passivierung erfolgt. Das Produkt wird anschließend entweder im Wirbelschicht-Trockner oder im Vakuum getrocknet und nach dem Trocknen durch ein 1,25mm-Sieb gesiebt.
Für das Endprodukt kann die im Beispiel 2 angeführte Carbonat-Phase Verwendung finden.

Für eine Dosis von 5 g enthaltend 7,5 mmol Magnesium werden 2960 Gew. teile der mit Magnesiumcarbonat passivierten Zitronensäure mit 1388 Gew. teilen Carbonat-Phase sowie 560 Gew.teilen Sorbit vermischt. Zu dieser Mischung werden nach Bedarf Süßstoffe sowie Aroma zugefügt. Diese Endmischung wird in Langsachets abgefüllt und zeigt hervorragende Eigenschaften im Hinblick auf die verzögerte Brausewirkung und auf eine gute Auflösung.

Mit dem System der verzögerten Brausewirkung kann man große lösliche Wirkstoffmengen mit einer geringen Menge an Brausegranulat auflösen, da die Granulatmischung mit dem Wirkstoff zu Boden sinkt und die dann relativ heftig einsetzende Reaktion mittels Durchwirbelung den Wirkstoff auflöst.

### Beispiel 7: Lactulose Brausegranulat

Für eine Dosis enthaltend 10 Gew.teile Lactulose werden 1,76 Gew.teile der mit Calciumcarbonat passivierten Zitronensäure, wie im Beispiel 1 beschrieben, eingesetzt und mit 1,23 Gew.teilen der Carbonat-Phase sowie 10 Gew.teilen Lactulose homogen vermischt und mit Süßstoffen und Aroma versehen.

Eine Dosis zu 13 g enthält 10 g Lactulose; das Produkt fällt beim Einbringen in Wasser zuerst zu Boden und beginnt dort nach 3 Sekunden heftig zu brausen, wobei die gesamte Lactulose aufgelöst wird. Es können auch 4 g der beiden Komponenten eingesetzt werden, womit ein noch besserer Brauseeffekt erzielt wird.

### Beispiel 8: Lactulose mit Elektrolyt-Ionen

Da es bei einem Diuretikum u.a. auch zu einem Verlust von Ionen, wie Calcium, Natrium, Kalium, Magnesium kommt, besteht die Möglichkeit, diese Ionen in das Carbonat-Granulat einzubauen und mit dem Produkt wieder zuzuführen.

In eine Dosis von 14 bis 15g, enthaltend 10 Gew.teile Lactulose, können etwa 90 bis 100 mg Calciumion, 100 mg Kaliumion, 90 mg Magnesiumion mit 270 mg Natriumionen eingebracht werden. Dazu werden in einem Mischer oder Granulator 2124 Gew.teile Zitronensäure grob kristallin mit 356 Gew.teilen Magnesiumcarbonat vermischt und auf 60°C erwärmt. Diese Mischung wird mit 250 Gew.teilen einer 50%igen wässrigen Zitronensäure benetzt, die darauf 10 Minuten lang homogen verteilt wird, wobei eine teilweise Reaktion und Passivierung erfolgt. Das Produkt wird anschließend getrocknet und nach dem Trocknen auf 1,5 mm gesiebt.

Daneben wird zur Herstellung einer verzögerten Carbonat-Phase folgendermaßen vorgegangen: 237 Gew.teile Calciumcarbonat, 830 Gew.teile Natriumhydrogencarbonat, 100 Gew.teile Natriumcarbonat und 267 Gew.teile Kaliumhydrogencarbonat werden homogen vermischt. Auf diese Mischung wird eine auf 130°C erhitzte Schmelze aus 142 Gew.teilen Polyethylenglycol, 42 Gew.teilen Keltrol, 2,8 Gew.teilen Aerosil, 12 Gew.teilen Miglyol 812 und 24 Gew.teilen Fumarsäure mikronisiert unter Rühren eingebracht und homogenisiert. Nach dem Abkühlen wird das Granulat auf eine Korngröße von 1,6 mm gesiebt.

Für das Fertigprodukt werden 2480 g der behandelten Zitronensäure, 1647 g des behandelten Carbonat-Granulates und 10 kg Lactulose sowie Süßstoffe und Aroma zugefügt und homogen vermischt. Das Granulat wird zu je 14,2 g in Sachets gefüllt. Es fällt beim Einbringen in Wasser zuerst zu Boden und beginnt dort nach 3 sec zu brausen, wobei die gesamte Lactulose in ca. 40 sec aufgelöst wird und eine geschmacklich angenehme Lösung ergibt.

### Beispiel 9: Acetylcystein 600 mg Brausegranulat

Es werden 700 Gew.teile der passivierten Zitronensäure entsprechend Beispiel 1, mit 600 Gew.teilen Acetylcystein und 700 Gew.teilen einer "verzögerten" Carbonat-Phase, die zusätzlich zum Calciumcarbonat und Natriumhydrogencarbonat noch 10 Gew.% Natriumcarbonat (bezogen auf das Natriumhydrogencarbonat) enthält, nach der Vorgangsweise von Beispiel 2 vermischt. Diese Mischung kann noch mit entsprechenden Süßstoffen und Aromen versehen werden.

2 bis 2,1 g dieser Mischung fallen beim Einbringen in Wasser zu Boden, beginnen nach ca. 2 - 3 sec zu brausen, und der Wirkstoff löst sich unter Brausen innerhalb von 30 sec vollständig auf.

### Beispiel 10: Paracetamol 1000 mg Brausegranulat

Sollen größere Mengen an schlecht benetzbarem Wirkstoff in der Formulierung enthalten sein, so empfiehlt es sich, zusätzlich zu den beiden wie in Beispiel 1 und 2 angeführten Phasen noch eine eigene Wirkstoff-Phase herzustellen.

Für eine Dosis von 5 g, in der 1 g Paracetamol enthalten ist, wird folgende Wirkstoff-Phase hergestellt: 1500 Gew.teile Mannit sowie 100 Gew.teile Natriumhydrogencarbonat werden homogen vermischt und auf 50°C aufgeheizt. Dann wird in den Mischer eine Lösung aus 40 Gew.teilen Polyvinylpyrrolidon K30 und 0,2 Gew.teilen Docusat-Natrium in 250 Gew.teilen Ethanol eingebracht und zur Benetzung gleichmäßig verteilt. Schliesslich werden 1000 Gew.teile Paracetamol zugefügt, worauf man die ganze Mischung nochmals mit einer Lösung aus 100 Gew.teilen Polyethylenglycol 6000 in 250 Gew.teilen Ethanol benetzt. Das Produkt wird vakuumgetrocknet und anschließend auf eine Korngrösse von 0,3 mm gesiebt.

Sodann wird die Granulat-Endmischung aus 2740 Gew.teilen der Paracetamol-Phase, 740 Gew.teilen der passivierten Zitronensäure und 1060 Gew.teilen der Carbonat-Phase hergestellt, die mit 216 Gew.teilen Sorbit homogen vermischt werden; dazu werden noch 60 Gew.teile Aspartam sowie 83 Gew.teile Aroma und weiters 100 Gew.teile einer Mannit/Simethicon-Phase zugefügt. 5 g dieses Produktes abgefüllt in Sachets fallen beim Einbringen in Wasser zu Boden, brausen anschließend auf und durchwirbeln das Paracetamol homogen in der Lösung.

Mit diesem System können auch Pflanzenextrakt-Brausegranulate hergestellt werden, wobei es bei einzelnen Extrakten erforderlich sein kann, eine eigene Phase für den Pflanzenextrakt herzustellen. Es gibt aber eine Reihe von Pflanzenextrakten, die direkt mit den wie in Beispiel 1 und 2 beschriebenen Grundgranulaten gemischt werden können, um ein entsprechendes Auflösungsbild zu erhalten:

### Beispiel 11: Pflanzenextrakt-Brausegranulat:

350 Gew.teile Echinacea-Trocken-Extrakt werden mit 1400 Gew.teilen passivierter Zitronensäure sowie 980 Gew.teilen "verzögerter" Carbonat-Phase und 934 Gew.teilen Sorbit vermischt. Dazu werden die entsprechenden Süßstoffe sowie das Aroma zugefügt. Eine Dosis von 3,5 bis 3,6 g zeigt den erwünschten Effekt des Hineinfallens und Brausens mit einer Durchwirbelung von unten, wobei der Echinacea-Extrakt aufgelöst wird.

### Beispiel 12: Brausegranulat mit Aminosäure, Vitaminen, Calcium und Magnesium

Zur Herstellung der Säure-Phase werden 2400 Gew.teile kristalliner Zitronensäure sowie 50 Gew.teile kristalliner Ascorbinsäure mit 240 Gew.teilen Calciumcarbonat vermischt. Diese Mischung wird mit 160 Gew.teilen einer Mononatriumcitratlösung (Vorreaktionslösung) benetzt und anreagiert. Dazu können noch 174 Gew.teile Magnesiumoxid eingebracht werden, und man kann mit weiteren 120 Gew.teilen derselben Lösung benetzen, wobei das Magnesiumoxid anreagiert.

Es wird weiters eine alkalische Natriumhydrogencarbonat-/Natriumcarbonat-Phase hergestellt, wobei zunächst 300 Gew.teile Natriumhydrogencarbonat grob kristallin mit 100 Gew.teilen Natriumcarbonat vermischt werden. Auf dieser Mischung verteilt man 60 Gew.teile einer Schmelze aus Polyethylenglycol 6000, die auf 130°C erwärmt wurde.

Dieser Phase kann - um eine höhere Calciumdosierung zu erhalten - noch zusätzlich Calciumcarbonat zugefügt werden.

Für das Fertigprodukt werden 2856 Gew.teile der Säurephase sowie 460 Gew.teile der Alkali-Phase mit 500 Gew.teilen Argininaspartat sowie Vitaminen, Süßstoffen, Farbstoffen und Aroma vermischt. Eine Dosis von 4,3 g enthält dann 500 mg Argininaspartat, eine gewünschte Dosierung der B-Vitamine, Vitamin C und Vitamin E, sowie Calcium und Magnesium. Das Granulat fällt beim Einbringen in Wasser zu Boden und beginnt von dort zu brausen, wobei die löslichen Wirkstoffe innerhalb von 40 Sekunden durch den Brauseeffekt aufgelöst werden.

## Patentansprüche

1. Brausegranulat mit verzögerter Brausewirkung, bestehend aus wenigstens je einer sauren und einer unter Säureeinwirkung gasabspaltenden Komponente aus Alkalihydrogencarbonat-, Alkalicarbonat- und/ oder Erdalkalicarbonatpartikeln, sowie gegebenenfalls beigemengten Wirkstoffen, Aromen, Pflanzenextrakten, Vitaminen, Mineralstoffen, etc., wobei die Partikel der sauren Komponente mit - vorzugsweise 1 bis 30 Gew.% - wenigstens einer der folgenden Verbindungen - gegebenenfalls unter Teilreaktion - überzogen sind: Alkalicarbonat, Alkalihydrogencarbonat, Erdalkalicarbonat, Erdalkalioxid, Hydrokolloid, **dadurch gekennzeichnet, daß** die Partikel der gasabspaltenden Komponente mit 5 bis 15 Gew.-% Polyethylenglycol - bezogen auf die gasabspaltende Komponente -, bevorzugt Polyethylenglycol 6000, oder Mischungen aus Polyethylenglycolen überzogen sind.

2. Brausegranulat nach Anspruch 1, **dadurch gekennzeichnet, dass** die gasabspaltende Komponente mit einer Schmelze aus Polyethylenglycol überzogen ist, die zumindest ein Kolloid, insbesondere Guar Gum oder Xanthan, und/oder zumindest eine mikronisierte Säure, insbesondere Fumarsäure oder Adipinsäure, enthält, wobei die gegebenenfalls vorhandene mikronisierte Säure vorzugsweise in einer Menge von 10-30 Gew.% des Polyethylenglycols vorliegt.

3. Brausegranulat nach einem der vorhergehenden Ansprüche, **dadurch gekennzeichnet, dass** ausserdem wenigstens 50 Gew.% der Partikeln der sauren Komponente eine Korngrösse über 0.2 mm, insbesondere zwischen 0.4 und 0.7 mm, aufweisen.

4. Brausegranulat nach einem der vorhergehenden Ansprüche, **dadurch gekennzeichnet, dass** die gasabspaltenden Komponenten bis zu 30 Gew.% Erdalkalicarbonat, Rest Natriumhydrogencarbonat, das zu 50-85 Gew.% mit einer Körnung von 0,1-0,4mm vorliegt, sowie gegebenenfalls Kaliumhydrogencarbonat in einer Körnung < 0.5mm und/oder Natriumcarbonat in einer Körnung von 0.07-0.2mm, enthalten.

5. Brausegranulat nach einem der vorhergehenden Ansprüche, **dadurch gekennzeichnet, dass** es als Wirkstoff Magnesium enthält, insbesondere Magnesiumoxid, Magnesiumcarbonat und/oder Trimagnesiumdicitrat, in einer Menge von bis zu 30 % der sauren Komponente.

6. Brausegranulat nach einem der vorhergehenden Ansprüche, **dadurch gekennzeichnet, dass** es zum Ausgleich der Ionen-Verluste zusätzliche Elektrolyte enthält.

7. Brausegranulat nach einem der vorhergehenden Ansprüche, **dadurch gekennzeichnet, dass** es einen wasserunlöslichen Wirkstoff, gegebenenfalls in einer eigenen Phase, enthält.

8. Brausegranulat nach einem der vorhergehenden Ansprüche, **dadurch gekennzeichnet, dass** trockene Pflanzenextrakte, insbesondere in einer eigenen Phase, gegebenenfalls mit wenigstens einem Antischaum- und/oder Benetzungsmittel behandelt, enthalten sind.

9. Brausegranulat nach einem der vorhergehenden Ansprüche, **dadurch gekennzeichnet, dass** zumindest ein wasserlöslicher Wirkstoff, insbesondere Lactulose, und/oder ein Vitamin, insbesondere Vitamin C, und/oder Spurenelemente enthalten sind.

10. Brausegranulat nach einem der vorhergehenden Ansprüche, **dadurch gekennzeichnet, dass** es in einem Sachet vorliegt, das zwischen 5 und 20, vorzugsweise zwischen 10 und 15 cm lang, und 6 bis 30, vorzugsweise zwischen 10 und 20 mm breit ist.

11. Verfahren zur Herstellung der gasabspaltenden Komponente eines Brausegranulats mit verzögerter Brausewirkung, **dadurch gekennzeichnet, dass** die Partikel der gasabspaltenden Komponenten mit einem Polyethylenglycolfilm überzogen werden, indem einer homogenen Mischung aus Alkaliund/oder Erdalkalicarbonaten bzw. -hydrogencarbonaten eine Schmelze aus Polyethylenglycol, die gegebenenfalls zumindest ein Kolloid und/oder eine mikronisierte Säure enthält, mit einer Temperatur von 120-160°C, bevorzugt tropfenweise, zugeführt wird, und diese mit den Alkaliund/oder Erdalkalicarbonaten gemischt und gegebenenfalls anschliessend getrocknet wird.

## Claims

1. Effervescent granules with delayed effervescent effect, consisting of at least one acid component and one component of alkali hydrogen carbonate, alkali carbonate, and/or alkaline-earth carbonate particles evolving gas under the action of acid, as well as of active substances, fragrances, plant extracts, vitamins, minerals etc. admixed as needed, the particles of the acid component being coated with - preferably 1 to 30 % by weight of - at least one of the following compounds - possibly while undergoing a partial reaction: alkali carbonate, alkali hydrogen carbonate, alkaline-earth carbonate, alkaline-earth oxide, hydrocolloid, **characterized in that** the particles of the gas-evolving component are coated with 5 to 15 percent by weight of polyethylene glycol - referred to the gas-evolving component -, preferably polyethylene glycol 6000, or of mixtures of polyethylene glycols.

2. Effervescent granules according to claim 1, **characterized in that** the gas-evolving component is coated with a melt of polyethylene glycol containing at least one colloid, particularly gum guar or xanthan, and/or at least one micronized acid, particularly fumaric or adipic acid, where the micronized acid when present preferably is present in amounts of 10 to 30 % by weight of the polyethylene glycol.

3. Effervescent granules according to one of the preceding claims, **characterized in that** in addition at least 50 % by weight of the particles of the acid component have a grain size above 0.2 mm, and particularly one between 0.4 and 0.7 mm.

4. Effervescent granules according to one of the preceding claims, **characterized in that** the gas-evolving components contain up to 30 % by weight of alkaline-earth carbonate, the remainder being sodium hydrogen carbonate which is present to the extent of 50 to 85 % by weight in a grain sizes between 0.1 and 0.4 mm, and where applicable potassium hydrogen carbonate in a grain size of <0.5 mm and/or sodium carbonate in a grain size of 0.07 to 0.2 mm.

5. Effervescent granules according to one of the preceding claims, **characterized in that** they contain magnesium as an active substance, particularly magnesium oxide, magnesium carbonate and/or magnesium dicitrate, in an amount of up to 30 % of the acid component.

6. Effervescent granules according to one of the preceding claims, **characterized in that** they contain additional electrolytes in order to compensate ion losses.

7. Effervescent granules according to one of the preceding claims, **characterized in that** they contain an active substance that is insoluble in water, where applicable forming its proper phase.

8. Effervescent granules according to one of the preceding claims, **characterized in that** dry plant extracts, particularly in the form of their proper phase, and where applicable treated with at least one antifoaming and/or wetting agent, are contained in them.

9. Effervescent granules according to one of the preceding claims, **characterized in that** at least one water-soluble active substance, particularly lactulose, and/or a vitamin, particularly vitamin C, and/or trace elements are contained in them.

10. Effervescent granules according to one of the preceding claims, **characterized in that** they are present in a sachet that has a length between 5 and 20, preferably between 10 and 15 cm, and a width between 6 and 30, preferably between 10 and 20 mm.

11. Process for the preparation of the gas-evolving component of effervescent granules with delayed effervescent effect, **characterized in that** the particles of the gas-evolving components are coated with a polyethylene glycol film by adding, preferably drop by drop, a melt of polyethylene glycol, which where applicable contains at least one colloid and/or one micronized acid, at a temperature of 120-160 °C to a homogeneous mixture of alkali and/or alkaline-earth carbonates or hydrogen carbonates, and **in that** this melt is mixed with the alkali and/or alkaline-earth carbonates and, where applicable, subsequently dried.

## Revendications

1. Granulés effervescents avec une effervescence retardée, constitués par au moins un composant acide et un composant générant un gaz par suite de l'action d'un acide, choisi parmi les particules d'hydrogénocarbonate de métal alcalin, de carbonate de métal alcalin et / ou de carbonate de métal alcalino-terreux, en mélange, le cas échéant, avec des substances actives, des arômes, des extraits végétaux, des vitamines, des matières minérales, etc., où les particules du composant acide sont recouvertes - de préférence à raison de 1 à 30 % en poids - par au moins un des composés suivants, le cas échéant en le ou les faisant réagir partiellement : carbonate de métal alcalin, hydrogénocarbonate de métal alcalin, carbonate de métal alcalino-terreux, oxyde de métal alcalino-terreux, hydrocolloïde, **caractérisés en ce que** les particules du composant générant un gaz sont recouvertes avec 5 à 15 % en poids de polyéthylène glycol, par rapport au composant générant un gaz, de préférence un polyéthylène glycol 6000, ou un mélange de polyéthylène glycols.

2. Granulés effervescents selon la revendication 1, **caractérisés en ce que** le composant générant un gaz est recouvert avec du polyéthylène glycol fondu, qui renferme au moins un colloïde, en particulier de la gomme de guar ou du xanthane et / ou au moins un acide micronisé, en particulier de l'acide fumarique ou de l'acide adipique, l'acide micronisé éventuellement présent étant utilisé, de préférence, en une quantité égale à 10 - 30 % en poids du polyéthylène glycol.

3. Granulés effervescents selon l'une des revendications précédentes, **caractérisés en ce que**, en outre, au moins 50 % en poids des particules du composant acide ont une granulométrie dépassant 0,2 mm et, de préférence, entre 0,4 et 0,7 mm.

4. Granulés effervescents selon l'une des revendications précédentes, **caractérisés en ce que** les composants générant un gaz contiennent jusqu'à 30 % en poids de carbonate de métal alcalino-terreux, le reste étant de l'hydrogénocarbonate de sodium, dont 50 - 58 % en poids ont une granulométrie de 0,1 - 0,4 mm, ainsi que, le cas échéant, de l'hydrogénocarbonate de potassium avec une granulométrie < 0,5 mm et / ou du carbonate de sodium avec une granulométrie de 0,07 - 0,2 mm.

5. Granulés effervescents selon l'une des revendications précédentes, **caractérisés en ce qu'**ils contiennent en tant que substance active du magnésium, en particulier de l'oxyde de magnésium, du carbonate de magnésium et / ou du dicitrate de tri-magnésium en une quantité allant jusqu'à 30 % du composant acide.

6. Granulés effervescents selon l'une des revendications précédentes, **caractérisés en ce que**, pour compenser la perte d'ions, ils contiennent des électrolytes additionnels.

7. Granulés effervescents selon l'une des revendications précédentes, **caractérisés en ce qu'**ils contiennent une substance active insoluble dans l'eau, le cas échéant dans une phase séparée.

8. Granulés effervescents selon l'une des revendications précédentes, **caractérisés en ce qu'**ils contiennent un extrait végétal sec, en particulier dans une phase séparée, le cas échéant avec au moins un agent antimousse et / ou un agent mouillant.

9. Granulés effervescents selon l'une des revendications précédentes, **caractérisés en ce qu'**ils contiennent au moins une substance active hydrosoluble, en particulier du lactulose et / ou une vitamine, en particulier de la vitamine C et / ou des oligo-éléments.

10. Granulés effervescents selon l'une des revendications précédentes, **caractérisés en ce qu'**ils sont placés dans un sachet qui a une longueur entre 5 et 20 et, de préférence, entre10 et 15 cm de longueur et une largeur entre 6 et 30 et, de préférence entre 10 et 20 mm.

11. Procédé de préparation du composant générant un gaz pour des granulés effervescents avec une effervescence retardée, **caractérisé en ce que** les particules du composant générant un gaz sont recouvertes d'un film de polyéthylène glycol, dans une opération où, à un mélange homogène de carbonates de métaux alcalins et /ou de carbonates de métaux alcalino-terreux et éventuellement d'hydrogénocarbonates de métaux alcalins, on ajoute un polyéthylène fondu, qui contient le cas échéant au moins un colloïde et / ou un acide micronisé, à une température de 120 - 160 °C, de préférence goutte à goutte, que le produit obtenu est mélangé avec des carbonates de métaux alcalins et / ou des carbonates de métaux alcalino-terreux et que le mélange est ensuite éventuellement séché.
